**Europäisches Patentamt**

**European Patent Office**

**Office européen des brevets**

(19)

(11) Veröffentlichungsnummer: **0 144 571**

**B1**

(12)

# EUROPÄISCHE PATENTSCHRIFT

(45) Veröffentlichungstag der Patentschrift: **23.11.88**

(51) Int. Cl.⁴: **A 61 F 7/02**

(21) Anmeldenummer: **84110929.1**

(22) Anmeldetag: **13.09.84**

(54) **Verfahren zur lokalen Wärmebehandlung mittels Packungen und Befestigungseinrichtung hierfür.**

(30) Priorität: **19.09.83 DE 3333778**

(43) Veröffentlichungstag der Anmeldung:
**19.06.85 Patentblatt 85/25**

(45) Bekanntmachung des Hinweises auf die Patenterteilung:
**23.11.88 Patentblatt 88/47**

(84) Benannte Vertragsstaaten:
**AT CH DE FR GB IT LI SE**

(56) Entgegenhaltungen:
**DE-A-2 444 611**
**DE-A-2 708 166**
**DE-C-2 611 928**
**US-A-3 801 995**

**DUDEN, Band 6, Seite 2840**

(73) Patentinhaber: **Haslauer, Paul, Moosstrasse 103,
A-5020 Salzburg (AT)**

(72) Erfinder: **Haslauer, Paul, Moosstrasse 103, A-5020
Salzburg (AT)**

(74) Vertreter: **Flach, Dieter Rolf Paul, Dipl.- Phys.,
Patentanwälte Andrae/Flach/Haug/Kneissl
Prinzregentenstrasse 24, D-8200 Rosenheim (DE)**

LIBER, STOCKHOLM 1988

**Beschreibung**

Die Erfindung betrifft ein Verfahren und eine Vorrichtung zum Anlegen von Wärmepackungen nach dem Oberbegriff des Anspruches 1 bzw. 11.

Wärmeanwendungen zählen zu sehr alten Therapieformen. Dabei sind prinzipiell zwei grundsätzlich verschiedene Methoden zu unterscheiden, nämlich einerseits die Verabreichung von heißen Bädern und andererseits die lokalbeschränkte Anwendung von heißen Packungen.

Unter Wärmepackungen versteht man in Fachkreisen eine örtliche Überwärmebehandlung mit der Möglichkeit, weitaus höhere Temperaturen als bei den oben erwähnten Bädern anzuwenden. Dies liegt darin begründet, daß bei Anwendung von Gesamtkörperbädern die Wärmebelastung insgesamt größer ist und die Grenzen dort gesetzt werden müssen, wo insbesondere auch bei älteren Menschen eine nicht mehr zumutbare Körperbelastung entsteht.

Demgegenüber können bei lokaler Anwendung Heißpackungen mit deutlich höheren Temperaturen angewandt werden, ohne dabei die Körpertemperatur allgemein zu steigern. Wünschenswert ist bei Heißpackungen auch ein direkter Kontakt vom Peloid zur menschlichen Haut.

In ständiger Praxis wird dabei der zu Behandelnde auf eine flache feste Liege gelegt, um danach die Heißpackungen gezielt an den lokal zu behandelnden Stellen aufzulegen. Um den Temperaturverlauf über längere Zeit zumindest ansatzweise konstant zu halten, werden wiederum auf den Heißpackungen, beispielsweise den Peloidpackungen Wärmeträger aufgelegt, die separat in einem Aufwärmofen auf eine gewünschte Temperatur erhitzt werden. Derartige Wärmeträger weisen erfahrungsgemäß ein Gewicht von 8 kg auf. Als nachteilhaft erweist sich dabei also zum einen allein der ständige Transport der schweren Wärmeträger vom Aufwärmofen zu der zu behandelnden Person. Als weiter nachteilhaft ergibt sich ferner, daß unter dem großen Gewicht der Wärmeträger häufig eine Peloidpackung an zumindest einer Stelle so durchgedrückt werden kann, so daß es aufgrund des nahen Kontaktes zwischen Wärmeträger zu einer lokalen Stelle der zu behandelnden Person zu einer Überhitzung auf der Haut kommen kann, was subjektiv als ein Art "Verbrennen" empfunden wird. Besonders problematisch ist aber vor allem, die Packung im Bereich der Lenden und Genick/Schulter so anzulegen, daß es auch nach Bewegungen des Patienten zu keinem Minderkontakt mit der Packung kommt.

Wird andererseits die Packung zu fest "verzurrt", so kann es zu einem unerwünschten Engegefühl bei der zu behandelnden Person kommen.

Zwar sind wesentliche Verbesserungen durch eine technische Neuerung gemäß der DE-PS-2 611 928 möglich geworden, indem zur lokalen Behandlung zwei Beutel in Anlage verwendet werden, nämlich eine Heißpackung, die beispielsweise mit Peloid gefüllt ist und einen zweiten darauf aufliegenden Beutel als Wärmespeicher. Die Anbringung dieser Packung im Bereich Genick/Schulter ist aber auch hier problematisch. Völlig ungelöst ist aber ferner bis heute, eine Heißpackung derart anzubringen, ohne daß die starre Lagerung des Patienten auf der Packungsliege zu einer erhöhten Muskelverspannung und zu Schmerzzuständen führt, und dies gerade bei Patienten mit einer Fehlhaltung, bei denen die Verabreichung von Heißpackungen häufig eher zu einer Verschlimmerung auf der Liege führen. Das Rückgrat und die Schulter sind wohl das komplizierteste Gelenksystem am menschlichen Körper. Sie stellen bei den Behandlungen mit Warmpackungen die Mehrzahl der Anwendungen. Wenn man mit einer heißen Peloidpackung an diese Gelenke herangehen will, so darf die Lagerung des Patienten während der Behandlung nicht außer Acht bleiben. Schließlich und endlich muß als nachteilhaft konstatiert werden, daß der Temperaturverlauf der verabreichten Packung nach der ersten Hälfte der Behandlungszeit doch stärker abfällt.

Demzufolge ist es Aufgabe der Erfindung, die Nachteile nach dem Stand der Technik zu überwinden und ein Verfahren zum Anlegen von Wärmepackungen ausgenommen zur therapeutischen Behandlting des menschlichen oder tierischen Körpers sowie eine Vorrichtung zur Durchführung eines Verfahrens zum Anlegen von Wärmepackungen zu schaffen, so daß die Anbringung der Packungen in jedem lokalen Bereich, insbesondere auch im Bereich Genick/Schulter in einer optimalen Schonhaltung bei einer zu behandelnden Person möglich ist, daß dies zu keiner Bewegungseinengung bei der zu behandelnden Person führt und andererseits auch ein Temperaturabfall bei den zur Anwendung gelangenden Packungen vermeidbar ist. Die Aufgabe wird bezüglich des Verfahrens erfindungsgemäß entsprechend den im kennzeichnenden Teil des Anspruches 1 und bezüglich der Vorrichtung entsprechend den im kennzeichnenden Teil des Anspruches 11 bzw. 13 angegebenen Merkmalen gelöst. Vorteilhafte Ausgestaltungen des beanspruchten Verfahrens und der Vorrichtung sind in den Unteransprüchen angegeben.

Durch die Erfindung wird eine wesentliche Verbesserung auf dem Gebiet der Verabreichung von lokalen Wärmepackungen realisiert. Erstmals ist es nämlich möglich, daß in allen lokalen Behandlungsbereichen, insbesondere auch im Bereich Genick/Schulter Wärmepackungen festanliegend angebracht werden können, ohne daß dies zur Beeinträchtigung einer Bewegungsfreiheit der zu behandelnden Person führt. Irgendwelche am Patienten festzuzurrenden Bänder oder Tücher sind nicht mehr notwendig. Vor allem wird erstmals durch die Erfindung auch bei der Verabreichung von Wärmepackungen sichergestellt, daß insbesondere auch bei zu behandelnden Personen mit Haltungsschäden noch zusätzliche Muskelverspannungen vermieden werden, da die Einnahme einer natürlichen Liegehaltung ohne Verspannungen möglich ist. Als weiterer Vorteil ergibt sich, daß durch die Anwendung eines umwälzbaren und aufheizbaren Mediums als Wärmespeicher der Temperaturverlauf in den Packungen, insbesondere auch in den Peloidpackungen über einen langen Behandlungszeitraum beliebig steuerbar sind, so daß zumindest ein Temperaturabfall auch über eine längere Behandlungszeit vermieden werden kann.

Dies wird dadurch ermöglicht, daß an der zu behandelnden Person Wärmepackungen angelegt und die verbleibenden Körperteile zumindest teilweise mit einer die Wärmeübertragung dämmenden Isolierschicht versehen werden. Eine feste Anlage wird dann mittels einer unter Druck stehender Andrückfolie erzielt, die mit einem als Wärmespeicher dienenden Medium in Kontakt steht.

In einer einfachen Ausführungsform nach Anspruch 2 wird dabei eine in einer ersten Betriebsstellung spannbare Ausdrückfolie verwendet, auf der die zu behandelnde Person liegend dann in ein als Wärmespeicher dienendes Medium in einer Wanne herabgesenkt wird, wobei alternativ oder ergänzend zu Anspruch 2 das als Wärmespeicher dienende Medium in der Wanne, beispielsweise aufgeheiztes Wasser direkt oder in einem entsprechenden Formballon eingegeben ist. Die Wandung des Formballons wirkt also hier entweder direkt als Andrückfolie oder gemeinsam mit der zusätzlich herabsenkbaren Auflagefolie. In allen Fällen aber wird über die Auflagefolie die Wärmepackung gleichmäßig und fest an die zu behandelnden lokalen Stellen am Körper angedrückt, wobei gleichzeitig durch das aufheizbare Medium als Wärmespeicher eine gewünschte Temperatursteuerung in den Wärmepackungen vorgenommen werden kann. Da die verbleibenden Teile des Körpers mit einer Isolierschicht abgedeckt sind, wird dort eine Wärmeübertragung vom Aufheizmedium im wesentlichen verhindert, bzw. eine mildere Überwärmung entsprechend der Dicke der Isolierfolie hervorgerufen, wodurch die lokale Wärmebehandlung ermöglicht wird.

Durch entsprechende Steuerung und Aufheizung des Wärmemediums kann zudem nach Anlegen der Packung sogar ein ansteigender Temperaturverlauf erzeugt werden, was bei der zu behandelnden Person als besonders angenehm empfunden wird, da ein sog. erster "Wärmeschock" vermieden wird. Gewisse Abwehrreaktionen des menschlichen Körpers werden dadurch vermieden.

Ansprüche 4 und 5 betreffen die Verwendung einer bevorzugten Isolierschicht mit mehreren Ausnehmungen, die vorzugsweise zur Aufnahme von Wärmepackungen an der zu der körperabweisenden Seite mit einer durchgängigen Folienbahn abgeschlossen sind.

Bei der vorgeschlagenen Isolierfolie lassen sich nach Anspruch 6 besonders günstig Moor- oder Peloidpackungen mit einer dickpastösen Schicht als Schlammfüllung verwenden, die praktisch auch unter Auflagendruck eine makroskopische Veränderung der Schichtdicke nicht aufweisen.

Alternativ dazu lassen sich aber auch Vorort spezifische dickpastöse Packungsmedien in gleichbleibender Schichtdicke in die entsprechenden Ausnehmungen der Isolierfolien eingeben, wobei dies durch Verwendung eines Rahmens mit Rastereinteilung nach Anspruch 8 und durch Verwendung einer zusätzlichen hauchdünnen Folie nach Anspruch 9 erleichtert wird. Durch die Rastereinteilung wird eine Höhenfixierung für einen gleichmäßigen Auftrag der dickpastösen Wärmepackungsschicht möglich, wobei durch Verwendung der hauchdünnen Folie nach einem Behandlungsvorgang der verwandte Moor- oder Schlammbrei sauber und leicht entfernbar ist.

Die Ansprüche 11 bis 25 betreffen eine erfindungsgemäße Vorrichtung zur Durchführung des Verfahrens zum Anlegen von Wärmepackungen. Danach ist eine Andrückfolie vorgesehen, über die an einer zu behandelnden Person angelegten Wärmepackungen und die an den verbleibenden Körperteilen aufgelegte Isolierschicht mit einem beheizbaren bzw. aufgeheizten und als Wärmespeicher dienenden Medium druckbeaufschlagt sind.

Bei einer Ausführungsform hängt zumindest während der Verabreichung der Wärmepackung die Andrückfolie in das Innere einer Wanne unter Druckbeaufschlagung der Unterseite der Andrückfolie durch das in der Wanne befindliche Medium. Die Handhabung wird noch vorteilhafter, wenn eine Hebe- und Senkvorrichtung für die Andrückfolie vorgesehen ist, wie dies beispielsweise in der bevorzugten Ausführungsform nach Anspruch 14 wiedergegeben ist. Zwar sind aus der DE-A1-244 611, der DE-A1-2 708 166 und der US-A-3 801 995 Vorrichtungen zum Anheben und Absenken von Körperbehinderten in eine Badewanne vorbekannt, mit denen aber abweichend von der vorliegenden Erfindung Wärmepackungen nicht entsprechend an einer zu behandelnden Person angelegt werden können.

Alternativ oder ergänzend zu einer während der Anwendung in die Wanne herabhängenden Andrückfolie kann das Wärmemedium auch in einem Formballon eingefüllt sein, dessen Wandung als Andrückfolie wirkt. In diesem Fall braucht die zu behandelnde Person also lediglich auf den Formballon aufgelegt zu werden. Vorteile ergeben sich aber gleichwohl bei zusätzlicher Verwendung einer anhebbaren und auf den Formballon absenkbaren Andrückfolie.

Ferner hat es sich bei einer bevorzugten Weiterbildung der Erfindung nach Anspruch 17 als günstig erwiesen, zusätzlich zwei unter Druck ausdehnbare Seitenballons zur Seitenanlage der Wärmepackung vorzusehen, wodurch gerade bei Krankheitsbildern im Bereich der Schulter und der Lende eine optimale Anlage von Schlamm- bzw. Moorpackungen gewährleistet ist.

Weitere Verbesserungen ergeben sich in den Weiterbildungen der Erfindung nach den Ansprüchen 18 bis 25, wobei günstige Ergebnisse der Behandlung sich insbesondere nach Anspruch 22 dann verwirklichen lasen, wenn die Wanne bzw. Formballons einschließlich der Seitenballons durch interne Trennwände in unterschiedliche Sektoren unterteilt sind, die gezielt mit einem dosierbaren Überdruck bei vorbestimmter Temperatur des Wärmemediums versorgt werden können.

Weitere Vorteile, Einzelheiten und Merkmale der Erfindung ergeben sich nachfolgend aus den anhand von Zeichnungen dargestellten Ausführungsbeispielen. Dabei zeigen im einzelnen:

Figur 1: eine schematische Teilansicht der verwandten Isolierschicht mit eingelegter Wärmepackung;

Figur 2: eine schematische Darstellung der Isolierschicht mit aufgelegter hauchdünner Folie und einem Rahmen mit Rastereinteilung zum Auftragen eines Moorbreies;

Figur 3: eine schematische Perspektivansicht der Vorrichtung zur Durchführung des Verfahrens;

Figur 4: eine schematische Stirnansicht der Vorrichtung nach Figur 2;

Figur 5: eine schematische Querschnittdarstellung eines weiteren Ausführungsbeispieles der erfindungsgemäßen Vorichtung;

Figur 6: eine schematische Seitenansicht der Vorrichtung nach Figur 5.

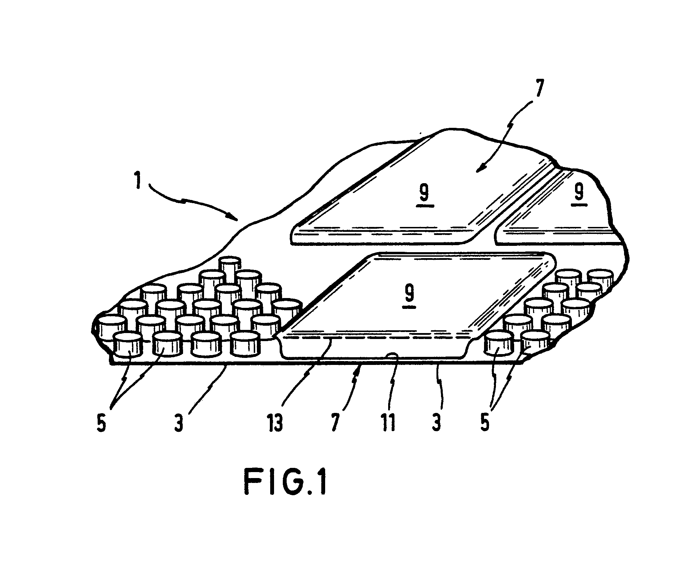

Im folgenden wird zunächst auf Figur 1 bezug genommen, in der eine Isolierschicht zur Anbringung von Packungen an einer zu behandelnden Person zur lokalen Wärmebehandlung ausschnittweise gezeigt ist. Die Isolierschicht 1 umfaßt dabei eine auf der Körper abweisen den Seite durchgängig verlaufende Folienbahn 3, auf der eine Vielzahl von Noppen 5 mit einer Dicke beispielsweise von 5 bis 15 mm angebracht ist. Darüber hinaus sind in der ausschnittweise gezeigten Isolierschicht 1 ferner mehrere Ausnehmungen 7 eingebracht, in die Wärmepackungen 9 in entsprechender Größe eingelegt werden können. Die Wärmepackungen 9 bestehen beispielsweise aus Heilschlamm, Moor, Peloid etc. und weisen in Draufsicht im wesentlichen eine reckteckförmige Form auf, wobei die Dicke der Wärmepackungen 9 in etwa zumindest der Höhe der Noppen 5 bzw. der Dicke der Isolierschicht 1 entspricht. An der in Figur 1 unten auf der Folienbahn 3 aufliegenden Seite der Wärmepackungen 9 besteht diese aus einer dichten Kunststoffolie 11, während auf der gegenüberliegenden Seite, also der dem Patienten zugewandten Seite ein Kunstfaservlies 13 vorgesehen ist, so daß die Befüllung in der Wärmepackung durch dieses Vlies hindurch treten und einerseits Sekrete aus der Haut herauslösen als auch andererseits wasserlösliche Wirkstoffe aus der Befüllung, beispielsweise den Peloid an die Hautoberfläche durchdiffundieren kann.

Die Füllung in der Wärmepackung 9 besteht dabei aus einer gleichmäßigen dickpastösen Schicht, um sicherzustellen, daß bei der Belastung durch den Patienten die Schichtdecke im wesentlichen im makroskopischen Maßstab betrachtet nicht verändert wird, so daß eine lokale Überhitzung vom später noch zu erläuternden Wärmespeicher ausgeschlossen werden kann. Die dickpastöse Konsistenz mit einer gleichmäßigen Schichtdecke ist für den gleichmäßigen Wärmeeinstrom von großer Bedeutung. An den Rändern ist das Vlies 13 mit der Kunststoffolie 11 verschweißt.

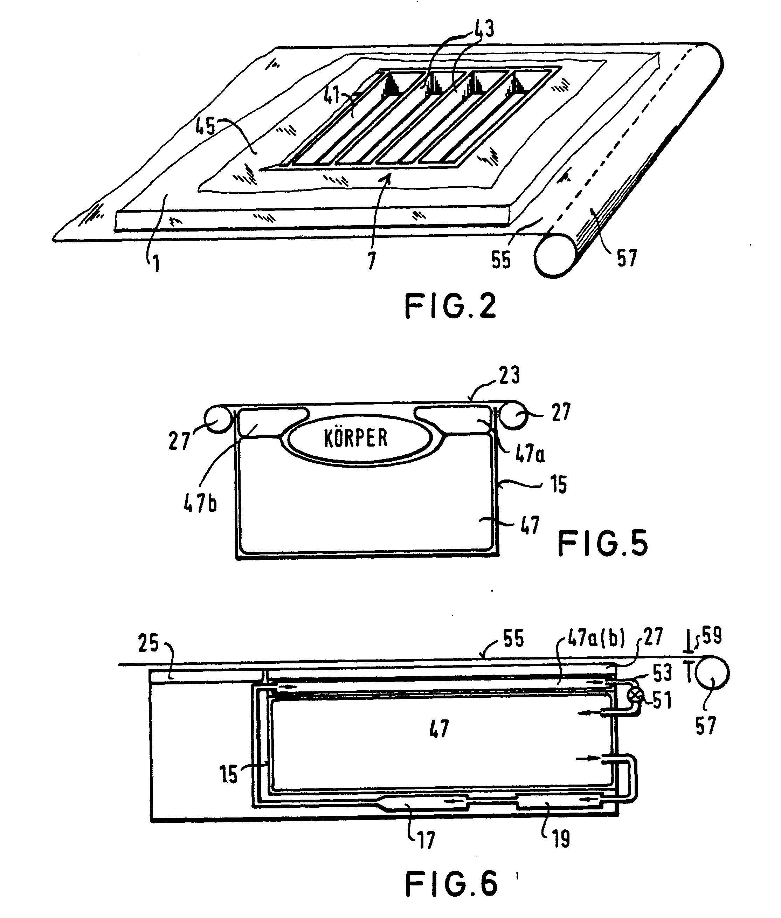

In Figur 2 ist eine Abwandlung zu Figur 1 für den Fall gezeigt, daß bei Anwendung des Verfahrens nicht eine vorgefertigte Peloid- bzw. Wärmepackung 9, sondern beispielsweise ortsgebundene spezielle und nicht verpackte Moor- oder Schlammedien angewandt werden sollen.

Dazu ist im Ausführungsbeispiel nach Figur 2 vorgesehen, daß über die Isolierschicht 1 zunächst eine hauchdünne Folie 45 gelegt wird, deren Abmessung mindestens der Ausnehmung entspricht. Danach wird ein viereckiger Rahmen 41 mit Rastereinteilung 43 in die Ausnehmung 7 eingedrückt, der dann die hauchdünne Folie an den Ausnehmungsrändern fest und gespannt eingedrückt hält. Mittels dieses Rahmens wird dann mit einem großen Spatel das Packungsmedium verteilt. In diesem Ausführungsbeispiel rutscht das Packungsmedium auf der Folie dann nicht mehr weg, wobei die Ränder scharf abgegrenzt und die Packungsdichte durch die Führung des Spatels über die Rastereinteilung gleichmäßig erfolgt.

Wenn der Rahmen vor dem Einlegen in ein Gleitmittel z. B. Wasser gelegt wird, bleibt an den Rändern und an der Rasterunterteilung das Packungsmaterial nicht kleben, sondern läßt sich gut abheben. Nach Entfernung des Rahmens werden die verbleibenden Streifen im Packungsfeld mit einem Spatelzug glattgestrichen. Der Rahmen läßt sich insbesondere deshalb leicht herausheben, ohne daß die verwandte Wärmepackung mitabgehoben wird, da die Rastereinteilung eine geringere Höhe als der Seitenrahmen aufweist, so daß die einzelnen Raster nicht auf der Folie 45 aufliegen. Das dann unterhalb der Rastereinteilung 43 durchgehende Wärmepackungsmedium wird dann - wie erwähnt - beim Abheben des Rahmens nicht mitgenommen.

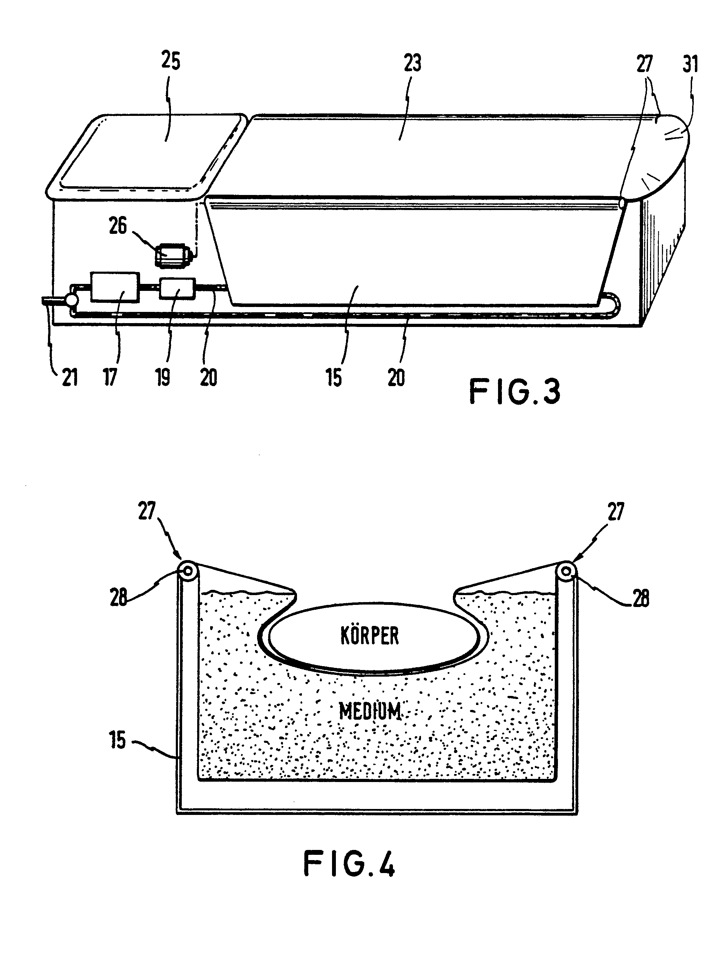

Im folgenden wird noch auf die Vorrichtung nach Figur 3 und 4 eingegangen, in denen in perspektivischer Darstellung bzw. Stirnschnittdarstellung eine Wanne 15 gezeigt ist, die beispielsweise bis in 3/4 Höhe mit einem wärmetragenden flüssigen Medium befüllt ist, das beispielsweise selbst aus einem Peloidbad oder aber auch aus einem Wasserbad oder einem anderen Medium bestehen kann. Die Wanne 15 ist ferner mit einem Wärmeaustauscher bzw. am Aufheizgerät 17 und einer Pumpe 19 versehen, um das wärmetragende Medium im Inneren ständig über Leitungen 20 umzuwälzen und auf eine beliebige Temperatur aufzuheizen bzw. abzukühlen. Über ein Anschlußstück 21, als Vor- und Rücklauf ausgebildet, kann mittels isolierten Schläuchen ein außerhalb des Beckens z. B. für Kniepackungen verwendeter Wärmeträgerbeutel im gleichen Heizkreis mitbeheizt werden.

Ferner umfaßt die Wanne 15 eine in der Figur 2 horizontal gespannt gehaltene Auflage- oder Andrückfolie 23 und an der einen Stirnseite am oberen Rand eine vorzugsweise gepolsterte Ablagefläche 25 auf. Wie ferner aus Figur 2 und 3 hervorgeht, ist die Andrückfolie 23 in einer Betriebsstellung durch die beiden seitlich längs der Wanne vorgesehenen Hebe- und Senkvorrichtung 27 im gespannten Zustand gehalten. Die Hebe- und Senkvorrichtung 27 kann dabei aus einer über einen Motor 26 antreibbaren Aufwickelstange 28 am oberen Seitenrand der Wanne bestehen. Möglich ist aber auch, daß an dem Seitenlängsrand der Andrückfolie 23 eine Hebe- und Senkvorrichtung beidseitig der Wanne direkt ansetzt.

Nachfolgend wird auf die Anbringung der Wärmepackung näher eingegangen.

Eine zu behandelnde Person legt sich zunächst auf die im wesentlichen horizontal fest gespannt gehaltene weiche Auflage- und Andrückfolie 23, wobei in diesem Zustand die Andrückfolie 23 mit der am Fußende angebauten gepolsterten Ablage 25 für die Beine vom Knie ab abwärts in einer Ebene liegt.

In diesem Zustand kann nach der Packungsbehandlung auch gleich die üblicherweise verordnete Massage oder andere Behandlung erfolgen.

Beim Anlegen einer Packung im Bereich Lenden, Rücken, Schulter und Genick wird zunächst eine oder mehrere Wärmepackungen 9 in die entsprechenden Ausnehmungen 7 einer Isolierschicht 1 mit oben aufliegenden Kunstfaservlies 13 eingelegt. Diese Wärmepackungen bestehen beispielsweise aus Einmal-Naturmoorpackungen. Die so mit den Wärmepackungen 9 ausgelegte Isolierschicht 1 wird dann auf der gespannt gehaltenen Andrückfolie 23 so ausgerichtet, daß beim Hineinlegen der zu behandelnden Person die Wärmepackungen an den zu behandelnden lokalen Korperstellen zu liegen kommen. In Bereichen, wo nicht behandelt wird, wird mit einer weiteren Isolierschicht 1, bzw. Decken oder ähnlichen oder beispielsweise im Bereich des Kopfes auch mit einem dünnen Kissen isoliert. Nachdem sich der Patient auf die Wärmepackungen 9 und auf die Isolierschicht 1 gelegt hat, wird sodann die Hebe- und Senkvorrichtung betätigt, so daß der Patient mit den Packungen und der Isolierschicht 1 in das wärmetragende Medium in der Wanne 15 hinabgesenkt wird. Beim Eintauchen in das flüssige Medium schmiegt die Andrückfolie 23 die Wärmepackungen 9 gut aber nicht einengend an die zu behandelnde Person an. An den Stellen, wo die Einmalpackung angelegt ist, steigt dann die Temperatur bis auf die gewünschte Behandlungstemperatur, die ja durch eine thermostatische Temperaturregelung über den Wärmetauscher einstellbar ist. Da durch die ständige Umwälzung und Aufwärmung das wärmetragende Medium nach einem anfangs eingestellten Temperaturanstieg auf einer gewünschten Temperatur gehalten werden kann, tritt ein langsames Abkühlen der Wärmepackungen selbst - wie nach dem Stand der Technik - nicht ein, weil ständig warme Flüssigkeit an die nur durch die Folienbahn 3 getrennt in der Isolierschicht 1 eingelegten Wärmepackung herangeführt wird. An den mittels der Isolierschicht 1 isolierten Stellen wird demgegenüber eine deutlich geringere Wärmeübertragung vom flüssigen Medium in der Wanne auf die zu behandelnde Person übertragen. Welche Haltung oder Abmessungen die zu behandelnde Person auch hat, sie schwebt in dem warmen wärmetragenden Medium. Gegebenenfalls können sogar leichte Bewegungsübungen während der Packungsbehandlung durchgeführt werden, ohne daß dies eine nachteilhafte Auswirkung auf die Haftung der Packung an der Körperoberfläche hat. Bis z. B. einschließlich des Gesäßes liegt die zu behandelnde Person etwas vertieft, während die Beine auf der gepolsterten Auflage bzw. Ablage 25 ruhen. Diese Hochlagerung der Beine ist aber erwünscht.

Nach Ablauf der Behandlungszeit wird die Hebe- und Senkvorrichtung 27 wieder betätigt, indem über die erwähnte Aufrollvorrichtung die Ablage- und Andrückfolie 23 wieder in eine horizontal gespannt gehaltene Stellung gebracht wird. Die Folie hebt also den Patienten mit der Packung aus dem wärmetragenden Medium heraus. Nach dem Aufsetzen können die behandelten Körperstellen von Schweiß und Peloidresten gereinigt. Einlagen aufgelegt und sodann die geschilderte Vorrichtung als Nachruhelager oder als Liege für die nachfolgende Massage verwendet werden. Ein Platzwechsel mit Ankleiden, verbunden mit der Gefahr einer Verkühlung wird hierdurch vermieden.

Sofern gleichzeitig oder alternativ noch eine lokale Wärmebehandlung im Bereich Knie, Bein oder Fuß durchgeführt werden soll, kann hierzu noch ein um das Bein herum legbarer Wärmeschlauch an die Vorrichtung angeschlossen werden, wobei das wärmespendende flüssige Medium im Becken über eine Pumpe durch diesen anlegbaren Wärmebeutel hindurch gepumpt wird. und dieser Beutel z. B. mit einer Einmal-Peloidpackung überlagert appliziert wird. Dadurch ergibt sich ein ähnlicher, ansteigender Temperaturverlauf wie im Bereich der Rücken-Schulterpackung.

Nur der Vollständigkeit halber soll noch erwähnt werden, daß an den Stirnseiten der Wanne die erwähnte Andrückfolie 23 noch überstehende Randbereiche 31 aufweist, so daß auch im herabgelassenen Zustand der Andrückfolie an den Stirnseiten kein flüssiges Medium auf die Oberseite der Andrückfolie 23 strömen kann.

In den Figuren 5 und 6 ist eine Abwandlung der Vorrichtung zu den Figuren 3 und 4 gezeigt. Danach ist in der Wanne 15 das als Wärmespeicher dienende Medium nicht direkt, sondern in einem Formballon 47 eingebracht, das über einen Zu- und Ablauf über einen Wärmetauscher bzw. über eine Heizung 17 und eine Pumpe 19 mit dem Medium umwälzend beheizt wird. Die Ballonwand wirkt in diesem Falle schon als Andrückfolie, so daß eine zu behandelnde Person mit den angebrachten Wärmepackungen und der Isoliermatte lediglich auf den Formballon aufgelegt werden muß. Durch den sich verteilenden Druck werden dann die Wärmepackungen bereits am Körper anliegend gehalten.

Zusätzlich können noch zwei weitere Seitenballons 47a und 47b vorgesehen sein, um insbesondere bei Krankheitsbildern im Bereich der Schulter und der Lende eine noch verbesserte seitliche Anlage der Wärmepackung zu erzielen.

Die Seitenballons 47a und 47b werden ebenfalls über die Pumpe und die Heizung mit Wärmemedium versorgt, wobei am Rücklauf ein Rücklaufdrosselventil 51 vorgesehen ist. Das Rücklaufdrossenventil 51 kann so geregelt werden, daß das Rücklaufdrosselventil im geöffneten Zustand das wärmetragende Medium lediglich hindurchfließen läßt, so daß die Seitenballons praktisch drucklos in sich zusammenfallen. Bei entsprechendem Schließen des Rücklaufdrosselventils entsteht vor diesem ein Überdruck, so daß aufgrund des steigenden Drucks die Seitenballons anschwellen und die Wärmepackung entsprechend einstellbar fest am Körper einer zu behandelnden Person anliegend halten. Bei gegebenem Durchlaufwiderstand kann dies auch durch Erhöhung der Pumpenleistung geregelt werden.

Im gezeigten Ausführungsbeispiel führt dabei die Rücklaufleitung 53 von den Seitenballons zunächst in den unteren Formballon und von dort über eine weitere Leitung zur Pumpe und Heizung. Möglich ist aber auch eine Verzweigungsleitung nach dem Rücklaufdrosselventil, um das ausströmende Wärmemedium aus dem Seitenballon 47a und 47b direkt zur Pumpe und Heizung zu transportieren.

Auch bei dieser Ausführungsform unter Verwendung eines Formballons und möglicherweise zusätzlicher Seitenballons, deren Ballonwandung bereits als Andrückfolie wirken, kann zusätzlich noch eine separate Andrückfolie 23 vorgesehen sein, die entsprechend dem Ausführungsbeispiel nach Figuren 2 und 3 über eine Hebe- und Senkvorrichtung 27 zwischen einer angehobenen gespannten und einer abgesenkten Betriebsstellung verstellt werden kann.

Abschließend sei noch angemerkt, daß sowohl der untere Formballon als auch die Seitenballons noch durch zusätzliche interne Trennwände in entsprechend separat mit Wärmemedium durchströmende Sektoren unterteilt sein können, um eine ganz gezielte Wärme- und/oder mechanische Behandlung zu ermöglichen. In dem jeweiligen ausgewählten Segment kann auch hier der Auslauf gedrosselt werden, so daß gezielt im jeweiligen Segment ein dosierbarer Überdruck entsteht, um eine bestimmte Packung in einem Bereich beispielsweise seitlich genau den Körperabmessungen entsprechend und vom Therapeuten steuerbar anzudrücken.

Abschließend wird nochmals auf die Figur 2 und 6 Bezug genommen, in denen die Verwendung eines zusätzlichen Vliesstoffes 55 näher erläutert ist.

Nach dem Einstreichen des Packungsmediums in die Ausnehmung 7 der Isolierschicht 1 wird eine durchlässige und z. B. auch saugfähige Bahn des Vliesstoffes 55 über die gesamte Länge der Isolierschicht 1 gezogen und abgetrennt, wobei die Breite des Vliesstoffes 55 vorzugsweise noch breiter ist als die Isolierschicht I. Dort wo sich das Packungsmedium befindet, bleibt das Vlies durchlässig, um den Materialfluß zwischen der Wärmepackung, beispielsweise dem Peloid und der menschlichen Haut zu ermöglichen.

Die Verwendung dieses Vliesstoffes weist auch den Vorteil auf, daß die beispielsweise aus Peloid bestehende Wärmepackung nicht auf der Haut festkleben bleibt. Der Patient muß von daher nicht sofort nach der Wärmebehandlung geduscht werden. Der durch die Behandlung intensiv überwärmte Körper wird dadurch auch nicht abrupt abgekühlt. Neben der Wärmepackung 9 wirkt der auf der Isolierschicht 1 aufgelegte Vliesstoff 55 nach Art eines Lakens, wodurch also auf einfache Art und Weise in einem Arbeitsgang gleichzeitig die Peloidschicht abgedeckt und außerhalb der Packung ein saugfähiges Wäschelaken aufgezogen werden kann. Bei Verwendung von vorgefertigten und vorabgepackten Wärmepackungen 9 gemäß Figur 1 kann gleichwohl eine solch durchgängige Vliesstoffbahn verwendet werden, wenn dadurch immer noch ein ausreichender Kontakt beispielsweise zwischen dem verwandten Peloid und der Haut eines Patienten gewährleistet ist.

In Figur 6 ist für die Verwendung des erwähnten Vliesstoffes 55 ein Vliesstoffspender 57 in Form einer Rolle an der Stirnseite der Wanne 15 vorgesehen, und zwar zusätzlich noch mit einer Schneidvorrichtung 59. Bei einer Behandlung muß also lediglich die Vliesstoffbahn von dem Vliesstoffspender 57 heruntergezogen und mittels der Schneidvorrichtung abgetrennt werden.

**Patentansprüche**

1. Verfahren zum Anlegen von Wärmepackungen, ausgenommen zur therapeutischen Behandlung des menschlichen oder tierischen Körpers, insbesondere Schlammpackungen, bestehend aus Schlamm, Moor etc., die auf den zu behandelnden Körperstellen auflegbar und mit einem anliegenden Wärmespeicher in Kontakt bringbar sind, dadurch gekennzeichnet, daß an den zu behandelnden Körperteilen Wärmepackungen (9) angelegt und die verbleibenden Körperteile zumindest teilweise mit einer die Wärmeübertragung dämmenden Isolierschicht (1) versehen werden, und daß dann die Isolierschicht (1) und die Wärmepackungen (9) über eine Andrückfolie (23, 47) mit einem beheizbaren bzw. aufgeheizten und als Wärmespeicher dienenden, flüssigen, breiigen oder gasförmigen Medium druckbeaufschlagt werden, wodurch die Wärmepackungen (9) am Körper der zu behandelnden Person voll anliegend gehalten werden.

2. Verfahren nach Anspruch 1, dadurch gekennzeichnet, daß die Andrückfolie (23) in einer ersten Betriebsstellung im wesentlichen horizontal festspannbar ist, und daß nach Anlegen der Wärmepackungen (9) und der Isolierschicht (1) die zu behandelnde Person durch seitliches Nachlassen der Andrückfolie (23) in das als Wärmespeicher dienende Medium mit der Andrückfolie (23) als Zwischenschicht herabgesenkt wird.

3. Verfahren nach Anspruch 1 oder 2, dadurch gekennzeichnet, daß die zu behandelnde Person mit den Wärmepackungen (9) und der Isolierschicht (1) auf einen mit dem beheizbaren und als Wärmespeicher dienenden befüllten Formballon (47) aufgelegt wird, über den die Wärmepackungen (9) am Körper der zu behandelnden Person voll anliegend unter Druckbeaufschlagung gehalten werden.

4. Verfahren nach einem der Ansprüche 1 bis 3, dadurch gekennzeichnet, daß eine Isolierschicht (1) mit einer oder mehreren Ausnehmungen (7) verwendet wird, in die eine oder mehrere Wärmepackungen (9) mit entsprechender Größe einbringbar sind.

5. Verfahren nach Anspruch 4, dadurch gekennzeichnet, daß eine Isolierschicht (1) mit einer durchgängigen Folienbahn (3) auf der körperabweisenden Oberseite zur Einlage der Wärmepackung (9) verwendet wird.

6. Verfahren nach einem der Ansprüche 1 bis 5 mit einer Wärmepackung, bestehend aus einem mit Schlamm, Moor etc. gefüllten Beutel, der auf der körperzugewandten Seite vorzugsweise mit einer den Schlamm durchlassenden Vliesabdeckung versehen ist, dadurch gekennzeichnet, daß durch die Andrückfolie (23, 47) fixierte Wärmepackungen (9) mit einem flachen Beutel mit einer als derart dickpastösen Schicht als Schlammfüllung verwendet werden, so daß auch unter dem Auflagedruck einer zu behandelnden Person eine makroskopische Veränderung der Schichtdicke im wesentlichen unterbleibt.

7. Verfahren nach einem der Ansprüche 1 bis 5, dadurch gekennzeichnet, daß dickpastöses Packungsmedium in gleichbleibender Schichtdicke vor dem Anlegen der Wärmepackung (9) an den zu behandelnden Körperteilen in die Ausnehmungen (7) in der Isolierschicht (1) mit gleichbleibender Schichtdicke eingebracht wird.

8. Verfahren nach Anspruch 7, dadurch gekennzeichnet, daß zum Einbringen des Packungsmediums (9) in den Ausnehmungen (7) ein wieder entfernbarer, oben und unten offener Rahmen (41) mit Rastereinteilung (43) verwendet wird, dessen Rastereinteilung (43) vorzugsweise vor der durchgängigen Folienbahn (3) endet.

9. Verfahren nach Anspruch 6, dadurch gekennzeichnet, daß vor dem Einbringen des Packungsmediums (9) eine sich zumindest über die Größe der Ausnehmungen (7) erstreckende hauchdünne Folie (45) mit dem Rahmen (41) in die Ausnehmung (7) hineingedrückt wird.

10. Verfahren nach einem der Ansprüche 1 bis 9, dadurch gekennzeichnet, daß nach dem Einbringen der Wärmepackungen (9) in die Ausnehmungen (7) ein Vliesstoff (55) auf der körperzugewandtliegenden Seite der Isolierschicht (1) vorzugsweise diese völlig überdeckend aufgelegt wird.

11. Vorrichtung zum Anlegen von Wärmepackungen, insbesondere Schlammpackungen, bestehend aus Schlamm, Moor etc., die mit einem anliegenden Wärmespeicher in Kontakt bringbar sind, gekennzeichnet durch eine Andrückfolie (23, 47), über die die an den zu behandelnden Körperstellen anlegbaren Wärmepackungen (9) bzw. die an verbleibenden Körperstellen anlegbare Isolierschicht (1) mit einem beheizbaren bzw. aufgeheizten und als Wärmespeicher dienenden flüssigen, breiigen oder gasförmigen Medium druckbeaufschlagbar sind.

12. Vorrichtung nach Anspruch 11, dadurch gekennzeichnet, daß die Andrückfolie (23) auf der Oberseite einer mit einem als Wärmespeicher dienenden Medium befüllbaren Wanne (15) angeordnet ist, die mit einer aufgelegten Person in das Innere der Wanne (15) unter Druckbeaufschlagung der Unterseite der Andrückfolie (23) durch das in der Wanne (15) befindliche Medium herabhängt.

13. Vorrichtung nach Anspruch 11 oder 12, dadurch gekennzeichnet, daß für die Andrückfolie (23, 47) und eine darauf aufgelegte Person eine Hebe- und Senkvorrichtung vorgesehen ist, über die die Andrückfolie (23, 47) mit einer aufgelegten Person zwischen einer ersten angehobenen Ausgangsstellung und einer demgegenüber zweiten herabgesenkten Betriebsstellung unter Druckbeaufschlagung der Unterseite der Andrückfolie (23) durch das Wärmemedium verstellbar ist.

14. Vorrichtung nach Anspruch 12 oder 13, dadurch gekennzeichnet, daß die auf der Oberseite der mit einem als Wärmespeicher dienenden Medium befüllbaren Wanne (15) angeordnete Andrückfolie (23), die in einer ersten Betriebsstellung im wesentlichen horizontal festspannbar und in einer zweiten Betriebsstellung mit einer aufgelegten Person in das Innere der Wanne (15) unter Druckbeaufschlagung der Unterseite der Andrückfolie (23) durch das in der Wanne (15) befindliche Medium herablaßbar ist.

15. Vorrichtung nach Anspruch 14, dadurch gekennzeichnet, daß die Andrückfolie (23) auch stirnseitige Randbereiche (31) zur Verhinderung des Eindringens des Mediums auf die Oberseite der Andrückfolie (23) aufweist.

16. Vorrichtung nach einem der Ansprüche 11 bis 15, gekennzeichnet durch zumindest einen vorzugsweise in der Wanne (15) angeordneten und mit als Wärmespeicher dienenden Medium befüllbaren Formballon (47), dessen nachgiebige Ballonwandung als Andrückfolie wirkt.

17. Vorrichtung nach einem der Ansprüche 11 bis 16, dadurch gekennzeichnet, daß zumindest zwei mit als Wärmespeicher dienendem Medium befüllbare und unter Druck ausdehnbare Seitenballons (47a, 47b) zur Seitenanlage von Wärmepackungen (9) im Seitenbereich einer zu behandelnden Person vorgesehen sind, deren Ballonwände zumindest mittelbar als seitliche Andrückfolien dienen.

18. Vorrichtung nach einem der Ansprüche 11 bis 17, dadurch gekennzeichnet, daß vorzugsweise eine motorisch betriebene Hebe- und Senkeinrichtung (27) zum Straffen und Herablassen der Andrückfolie (23) vorgesehen ist.

19. Vorrichtung nach einem der Ansprüche 11 bis 18, dadurch gekennzeichnet, daß zur Temperatursteuerung des durch die Wanne (15) bzw. den mindestens einen Formballon (47, 47a, 47b) hindurchströmenden Mediums eine Umwälzanlage (19) und eine Aufheizvorrichtung (17) vorgesehen ist.

20. Vorrichtung nach Anspruch 19, dadurch gekennzeichnet, daß bei zumindest beiden Seitenballons (47a, 47b) in ihrer Rücklaufleitung (49) ein Rücklaufdrosselventil (51) eingebaut ist.

21. Vorrichtung nach Anspruch 19, dadurch gekennzeichnet, daß bei gegebenem Rücklaufwiderstand zumindest in den Rücklaufleitungen (53) der Seitenballons (47a, 47b) die Leistung der Umwälzanlage (Pumpe 19) regelbar ist.

22. Vorrichtung nach Anspruch 19, 20 oder 21, dadurch gekennzeichnet, daß die Rücklaufleitung (53) von den Seitenballons (47a, 47b) in den unteren Formballon (47) führt, dessen Austrittsöffnung (55) mit der Umwälz- und Aufheizvorrichtung (19, 21) verbunden ist.

23. Vorrichtung nach einem der Ansprüche 11 bis 22, dadurch gekennzeichnet, daß ferner an einer Stirnseite der Wanne (15) eine horizontale Ablage (25) in Höhe des oberen Wannenrandes vorgesehen ist.

24. Vorrichtung nach einem der Ansprüche 11 bis 23, dadurch gekennzeichnet, daß ferner ein Vliesstoffspender (57), vorzugsweise an der Stirnseite der Wanne (15) vorgesehen ist.

25. Vorrichtung nach Anspruch 24, dadurch gekennzeichnet, daß ferner noch eine Schneidvorrichtung (59) zum Abschneiden der im Vliesstoffspender (57) lagerbaren Vliesstoffbahn (55) vorgesehen ist.

**Claims**

1. A method for the application of thermal packs, with the exception of those intended for the therapeutic treatment of the human or animal body, and more especially in the form of mud packs, consisting of mud, turf etc., which are able to be applied to the parts of the body to be treated and which are able to be placed in contact with an adjacent heat storing means, characterized in that thermal packs (9) are placed on the parts of the body to be treated and the remaining parts of body are provided at least partly with an insulating layer (1) preventing the transmission of heat, and in that the insulating layer (1) and the thermal packs (9) are acted upon by a pressure using a pressing foil (23 and 47) using a heatable or heated liquid, sludge-like or gaseous medium serving as a heat storage means so that the thermal packs (9) are held on the body of the person to be treated.

2. The method as claimed in claim 1, characterized in that the pressing foil (23) is able to be clamped in place in a first operation position in a generally horizontal position and in that after application of the thermal packs (9) and of the insulating layer (1) the person to be treated is lowered by lateral slackening off of the pressing foil (23) into the medium, serving as a heat storage means, with the pressing foil (23) as an intermediate layer.

3. The method as claimed in claim 1 or claim 2, characterized in that the person to be treated with the thermal packs (9) and the insulating layer (1) is placed on a shaped balloon (47) filled with the material serving as the heat storage medium and able to be heated, said balloon serving to hold the thermal packs (9) on the body of the person to be treated full adjacent thereto with the application of pressure.

4. The method as claimed in any one of the claims 1 through 3, characterized in that an insulating layer (1) is used with one or more pockets (7) into which one or more thermal packs (9) with a suitable size may be placed.

5. The method as claimed in claim 4, characterized in that an insulating layer (1) with a continuous foil sheet (3) is used on the top side facing away from the body for insertion of the thermal pack (9).

6. The method as claimed in any one of the claims 1 through 5 with a thermal pack, consisting of a bag filled with mud, turf etc., which on the side facing towards the body is preferably provided with a non-woven covering allowing passage of the mud, characterized in that the thermal packs (9) secured by the pressing foil (23 and 47) are used with a flat bag with a layer as a mud filling which is of such a thick paste-like consistency that even under the pressure of the person to be treated there is essentially no macroscopic change in the layer thickness.

7. The method as claimed in any one of the claims 1 through 5, characterized in that the thick, paste-like pack medium is placed with an even layer thickness prior to application of the thermal packs (9) on the parts of the body of the person to be treated in the pockets (7) in the insulating layer (1) with an even layer thickness.

8. The method as claimed in claim 7, characterized in that for insertion of the pack medium (9) in the pockets (7) use is made of a removable frame (41) which is open at the top and bottom with a grid (43), the grid (43) preferably ending short of the continuous foil sheet (3).

9. The method as claimed in claim 8, characterized in that prior to the insertion of the pack medium (9) an extremely thin foil (45) extending at least over the size of the pockets (7) is pressed with the frame (41) into the pocket (7).

10. The method as claimed in any one of the claims 1 through 9, characterized in that after the insertion of the thermal packs (9) into the pockets (7) a non-woven material (55) is placed on the side, facing towards the body, of the insulating layer (1) so as to preferably cover the latter completely.

11. A device for the application of thermal packs, more especially mud packs, consisting of mud, turf etc., which are able to be placed in contact with an adjacent heat storage means, characterized by a pressing foil (23 and 47), by way of which the thermal packs (9) adapted to be placed on the parts of the body to be treated or the insulating layer (1) adapted to be laid on the remaining parts of the body, are able to be acted upon by pressure using a heatable or heated liquid, sludge-like or gaseous medium serving as a heat storage means.

12. The device as claimed in claim 11, characterized in that the pressing foil (23) is arranged on the top side of a tub (15) able to be filled with a medium serving as a heat storage means, which with the person placed thereon hangs down into the interior of the tub (15) with the action of pressure on the lower side of the pressing foil (23) by the medium in the tub (15).

13. The device as claimed in claim 11 or claim 12, characterized in that there is a lifting and lowering means for the pressing foil (23 and 47) and a person laid thereon, by means of which the pressing foil (23 and 47) with a person laid thereon may be moved between a first lifted starting position and a second lower operating position accompanied by the action of pressure on the lower side of the pressing foil (23) by the thermal medium.

14. The device as claimed in claim 12 or claim 13, characterized in that the pressing foil (23) arranged on the top side of the tub (15) able to be filled with a medium serving as a heat storage means in a first operational position is able to be pulled taut in a generally horizontal position and in a second operation position with the person laid thereon is able to be lowered into the interior of the tub (15) with the application under pressure on the lower side of the pressing foil (23) of the medium located in the tub (15).

15. The device as claimed in claim 14, characterized in that the pressing foil (23) also has end edge parts (31) to prevent the penetration of the medium to the top side of the pressing foil (23).

16. The device as claimed in any one of the claims 11 through 15, characterized by at least one shaped balloon (47) preferably arranged in the tub (15) and able to be filled with medium serving as a heat storage means, the soft wall of such balloon serving as the pressing foil.

17. The device as claimed in any one of the claims 11 through 16, characterized in that at least two lateral balloons (47a and 47b) able to be filled with the medium serving as a heat storage means and able to be expanded under pressure are provided for the lateral application of thermal packs (9) on the side part of a person to be treated the walls of such balloons serving at least indirectly as lateral pressing foil.

18. The device as claimed in any one of the claims 11 through 17, characterized in that the lifting and lowering means (27), which is preferably driven by a motor, is provided for tightening and lowering the pressing foil (23).

19. The device as claimed in any one of the claims 11 through 18, characterized in that for the control of the temperature of the medium flowing through the tub (15) and the at least one shaped balloon (47, 47a and 47b) there is a circulating means (19) and a heating up device (17).

20. The device as claimed in claim 19, characterized in that in the case of at least both lateral balloons (47a and 47b) a return choke (51) is provided in their return duct (49).

21. The device as claimed in claim 19, characterized in that at a given return resistance at least in the return ducts (53) of the lateral balloons (47a and 47b) the power of the circulating means (pump 19) is able to be adjusted.

22. The device as claimed in any one of the claims 19, 20 and 21, characterized in that the return duct (53) leads from the lateral balloons (47a and 47b) in the lower shaped balloon (47), whose outlet port (55) is connected with the circulating and heating means (19 and 21).

23. The device as claimed in any one of the claims 11 through 22, characterized in that furthermore at one end side of the tube (15) there is a horizontal rest (25) at the same height as the upper rim of the tub.

24. The device as claimed in any one of the claims 11 through 23, characterized in that furthermore a dispenser (57) for non-woven material is provided, preferably at the end side of the but (15).

25. The device as claimed in claim 24, characterized in that furthermore a cutting means (59) is provided for cutting off the non-woven sheet (55) able to be stored in the dispenser (57) of non-woven material.

**Revendications**

1. Procédé d'application de cataplasmes chauffants, à l'exception du traitement thérapeutique du corps humain ou animal, en particulier des cataplasmes fangeux constitués de boue, de limon, etc., destinés à être appliqués sur les parties à traiter du corps et à être mis en contact avec un accumulateur de chaleur adjacent, caractérisé en ce qu'il est d'abord appliqué des cataplasmes (9) sur les parties à traiter du corps ainsi qu'un matelas isolant (1) au moins partiellement sur les parties restantes du corps, en vue d'empêcher un transfert de chaleur vers celles-ci, et en ce que le matelas isolant (1) et les cataplasmes (9) sont ensuite soumis, par l'intermédiaire d'une toile de pression et de maintien (23, 47), à la pression d'un milieu liquide, pâteux ou gazeux chauffé et chauffant servant d'accumulateur de chaleur, en sorte que les cataplasmes (9) se trouvent maintenus complètement appliqués contre le corps de la personne à traiter.

2. Procédé suivant la revendication 1, caractérisé en ce que, dans une première position d'utilisation, la toile de pression et de maintien (23) peut être tendue substantiellement à l'horizontale et en ce qu'après application des cataplasmes (9) et du matelas isolant (1), la personne à traiter est descendue, avec la toile (23) en tant que couche intermédiaire, dans le milieu servant d'accumulateur de chaleur, en libérant latéralement la toile (23).

3. Procédé suivant la revendication 1 ou 2, caractérisé en ce que la personne à traiter est installée avec les cataplasmes (9) et le matelas isolant (1) sur un ballon préformé (47) rempli du milieu chauffant servant d'accumulateur de chaleur, en sorte que les cataplasmes (9) se trouvent maintenus complètement appliqués sous pression contre le corps de la personne à traiter.

4. Procédé suivant une des revendications 1 à 3, caractérisé en ce qu'il est utilisé un matelas isolant (1) comportant un ou plusieurs évidements (7) dans lesquels un ou plusieurs cataplasmes (9) de grandeur correspondante sont introduits.

5. Procédé suivant la revendication 4, caractérisé en ce qu'il est utilisé un matelas isolant (1) présentant une surface de toile (3) continue du côté supérieur opposé au corps, destinée à y placer les cataplasmes (9).

6. Procédé suivant une des revendications 1 à 5, utilisant un cataplasme chauffant consistant en une poche remplie de boue, de limon, etc. présentant, du côté dirigé vers le corps, de préférence une toile laissant passer la boue, caractérisé en ce qu'il est utilisé des cataplasmes (9) présentant une poche plane comportant une charge de boue formant une couche de pâte épaisse, fixés par la toile de pression et de maintien (23, 47), en sorte que même sous la pression exercée par le corps d'une personne à traiter une modification macroscopique de l'épaisseur de la couche ne se produit substantiellement pas.

7. Procédé suivant une des revendications 1 à 5, caractérisé en ce qu'il est introduit une pâte épaisse pour cataplasmes d'épaisseur de couche constante dans les évidements (7) du matelas isolant (1), avant l'application du cataplasme (9) sur les parties à traiter du corps.

8. Procédé suivant la revendication 7, caractérisé en ce qu'il est utilisé un cadre (41) amovible ouvert vers le haut et vers le bas et divisé en compartiments (43) se terminant de préférence avant la toile continue (3), en vue d'introduire la matière pour cataplasmes (9) dans les évidements (7).

9. Procédé suivant la revendication 8, caractérisé en ce qu'il est introduit dans l'évidement (7), en même temps que le cadre (41), une feuille (45) très mince de la grandeur au moins de l'évidement (7), avant d'y introduire la matière pour cataplasmes (9).

10. Procédé suivant une des revendications 1 à 9, caractérisé en ce qu'il est disposé une toile nappée (55) sur le matelas isolant (1), du côté dirigé vers le corps de la personne à traiter, de manière à recouvrir de préférence entièrement ledit matelas isolant (1), après introduction des cataplasmes (9) dans les évidements (7).

11. Dispositif d'application de cataplasmes chauffants, en particulier des cataplasmes fangeux constitués de boue, de limon, etc., pouvant être mis en contact avec un accumulateur de chaleur adjacent, caractérisé en ce qu'il comporte une toile de pression et de maintien (23, 47) par l'intermédiaire de laquelle les cataplasmes (9) appliqués sur les parties à traiter du corps et le matelas isolant (1) appliqué sur les parties restantes du corps sont soumis à la pression d'un milieu liquide, pâteux ou gazeux chauffé et chauffant servant d'accumulateur de chaleur.

12. Dispositif suivant la revendication 11, caractérisé en ce que la toile de pression et de maintien (23) est installée du côté supérieur d'une baignoire (15) remplie d'un milieu servant d'accumulateur de chaleur, de manière à pouvoir être descendue dans ladite baignoire (15) avec une personne installée sur ladite toile (23), en vue de soumettre le côté inférieur de ladite toile (23) à la pression du milieu se trouvant dans la baignoire (15).

13. Dispositif suivant la revendication 11 ou 12, caractérisé en ce qu'il est prévu un dispositif de montée et de descente de la toile de pression et de maintien (23, 47) et de la personne s'y trouvant installée, de sorte que la toile de maintien (23, 47) avec la personne s'y trouvant installée peut être déplacée depuis une première position initiale relevée vers une deuxième position d'utilisation abaissée, soumettant ainsi le côté inférieur de la toile (23) à la pression du milieu chauffant.

14. Dispositif suivant la revendication 12 ou 13, caractérisé en ce que la toile de pression et de maintien (23) installée du côté supérieur de la baignoire (15) remplie d'un milieu servant d'accumulateur de chaleur, peut être tendue substantiellement à l'horizontale dans une première position d'utilisation et peut être abaissée, avec une personne installée dessus, dans une deuxième position d'utilisation à l'intérieur de la baignoire (15), de manière à ce que le côté inférieur de ladite toile (23) se trouve soumis à la pression du milieu se trouvant dans la baignoire (15).

15. Dispositif suivant la revendication 14, caractérisé en ce que la toile de pression et de maintien (23) présente une extension (31) à ses extrémités afin d'éviter la pénétration du milieu contenu dans la baignoire (15) du côté supérieur de la toile (23).

16. Dispositif suivant une des revendications 11 à 15, caractérisé en ce qu'il comporte au moins un ballon préformé (47) installé de préférence dans la baignoire (15) et rempli d'un milieu servant d'accumulateur de chaleur, dont la paroi souple agit comme une toile de pression et de maintien.

17. Dispositif suivant une des revendications 11 à 16, caractérisé en ce qu'il est prévu au moins deux ballons latéraux (47a, 47b) contenant un milieu servant d'accumulateur de chaleur et expansibles sous pression, en vue de l'application latérale de cataplasmes (9) sur les côtés d'une personne à traiter, dont les parois servent indirectement au moins de toile de pression et de maintien.

18. Dispositif suivant une des revendications 11 à 17, caractérisé en ce qu'il est prévu de préférence un système de montée et de descente (27) à moteur, en vue de tendre et d'abaisser la toile de pression et de maintien (23).

19. Dispositif suivant une des revendications 11 à 18, caractérisé en ce qu'il est prévu système de circulation (19) et un dispositif de chauffage (17) en vue de commander la température du milieu injecté respectivement dans la baignoire (15) et dans un des ballons préformés (47, 47a, 47b) au moins.

20. Dispositif suivant la revendication 19, caractérisé en ce que, pour les deux ballons latéraux au moins, il est intégré une soupape d'étranglement (51) dans leur canalisation de retour (49).

21. Dispositif suivant la revendication 19, caractérisé en ce que le débit du système de circulation (pompe 19) peut être réglé en fonction d'une résistance de retour donnée au moins dans la canalisation de retour (53) des ballons latéraux (47a, 47b).

22. Dispositif suivant la revendication 19, 20 ou 21, caractérisé en ce que la canalisation de retour (53) des ballons latéraux (47a, 47b) débouche dans le ballon préformé inférieur (47), dont l'orifice de sortie (55) se trouve relié au système de circulation et de chauffage (19, 21).

23. Dispositif suivant une des revendications 11 à 22, caractérisé en ce qu'il est encore prévu une surface horizontale (25) à hauteur du bord supérieur de la baignoire (15), à une des extrémités de celle-ci.

24. Dispositif suivant une des revendications 11 à 23, caractérisé en ce qu'il est encore prévu un distributeur de toile nappée (57), de préférence à l'extrémité de la baignoire (15).

25. Dispositif suivant la revendication 24, caractérisé en ce qu'il est encore prévu un dispositif coupeur (59) destiné à couper la bande de toile (55) stockée dans le distributeur de toile nappée (57).

# FIG.1

FIG.2

KÖRPER

FIG.5

FIG.6

FIG.3

KÖRPER

MEDIUM

FIG.4